# EUROPEAN PATENT SPECIFICATION

(11) **EP 0 683 171 B1**
(45) Date of publication and mention of the grant of the patent: **23.12.1998**
(21) Application number: 94303637.6
(22) Date of filing: 20.05.1994
(51) Int. Cl.: C07H 19/073

(54) **A process for the preparation of beta-thymidine**
Verfahren zur Herstellung von Beta-Thymidine
Procédé pour la préparation de bêta-thymidine

(43) Date of publication of application: 22.11.1995
(73) Proprietor: Council of Scientific and Industrial Research, New Delhi 110 001 (IN)
(72) Inventor: Rama Rao, Alla Ventaka, Hyderabad Andhra Pradesh (IN); Gurjar, Mukund Keshao, Hyderabad Andhra Pradesh (IN); Lalitha, Sista Ventaka Sai, Hyderabad Andhra Pradesh (IN)
(74) Representative: Moon, Donald Keith

(56) References cited:
- EP-A- 0 351 126
- US-A- 4 914 233
- JOURNAL OF THE CHEMICAL SOCIETY, CHEMICAL COMMUNICATIONS, no.10, 21 May 1994, LETCHWORTH GB pages 1255 - 1256 A.V.RAMA RAO ET AL. 'Discovery of a Novel Route to Beta-Thymidine: A Precursor for Anti-AIDS Compounds.'

## Description

The invention relates to an improved process for the preparation of beta-thymidine. Beta-thymidine prepared by the process of the present invention has the formula (II). 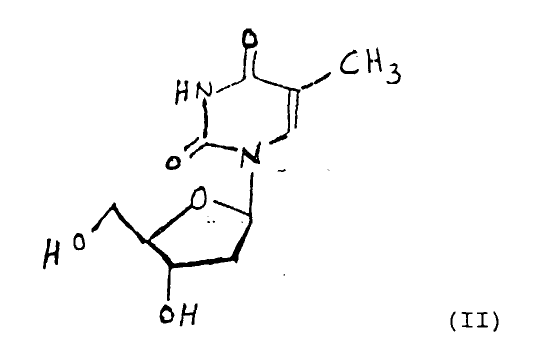 Beta-thymidine is an important intermediate for the preparation of dideoxynucleosides. Dideoxy-nucleosides of the formula (I) 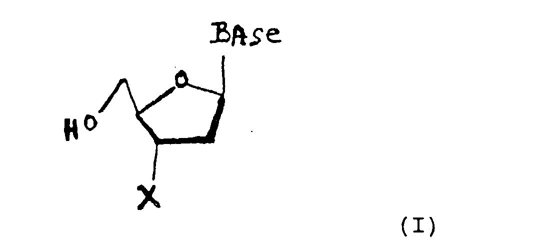 where X represents hydrogen or N₃, base represents thymine, cytosine or hypoxanthine are of current interest because of their clinical use in the treatment of acquired immuno deficiency syndrome (AIDS).

AZT of the formula (I) where X represents N₃ and base represents thymine, is the most active anti-AIDS agent for which many synthetic routes have been reported (R. Benhaddou et al, Bull, Soc. Chim. Fr., 1991 (Jan-Feb), 108-11; J.D. Wilson, US Pat. No. 4,921,950; Wellcome Foundation Ltd. Jp. Pat. No. 63,255,295; C. K. Chu, PCT Int. Appl. No. 9001,492; M.R. Almond et al, US Pat. No. 4,916,218).

The commercially useful route for the preparation of AZT uses beta-thymidine of the formula (II) as the starting material. (O. Szabolcs et al, Hung. Teljes, HU 48,901; S. Czernecki et al, Eur. Pat. No. 427,587; J.L. Rideout et al, Eur. Pat. No. 199,451; F.Y. Shealy et al, US Pat. No. 4,730,001; J.N. Freskos et al, US Pat. No. 4,914,233).

In the US Patent No. 4,914,233 a process has been disclosed for the synthesis of beta-thymidine in which a mixture of alpha and beta anomers of tetraacetylribofuranose of the formula (IV) 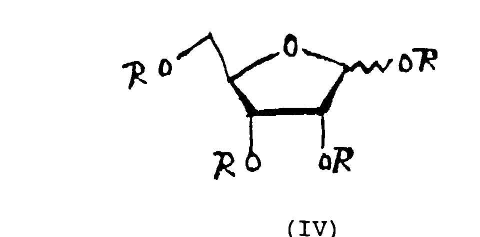 where R represents acetyl group is converted selectively to the desired beta-thymidine. The process involves the following steps (a) converting a mixture of alpha and beta anomers of tetra-O-acetylribofuranose to tri-O-acetyl-beta-ribothymidine (b) converting tri-o-acetyl-beta-ribothymidine to beta-ribothymidine (c) converting the beta-ribothymidine to 2, 2'-anhydro-beta-thymidine (d) converting 2, 2'-anhydro-Beta-thymidine to 2'-halo-2'deoxy-5-methyluridine and (e) converting 2'-halo-2'deoxy-5-methyluridine to beta-thymidine.

The mixture of alpha and beta anomers can be prepared by any suitable methods such as by converting lower alkyl ribofuranoside to the tetra-O-acetyl ribofuranose mixture. The lower alkyl ribofuranosides may in turn be prepared by various known methods. The desired method may make use of D-ribose as the starting material which is converted to the lower alkyl ribofuranoside.

The common way of preparing AZT from thymidine involves the preparation of 2, 3'-anhydro derivative followed by the displacement reaction with sodium azide to furnish AZT.

Accordingly the present invention provides an improved process for the preparation of beta-thymidine of the formula (II) which comprises:
(a) preparing the deprotected xylothymidine of the formula (IX) 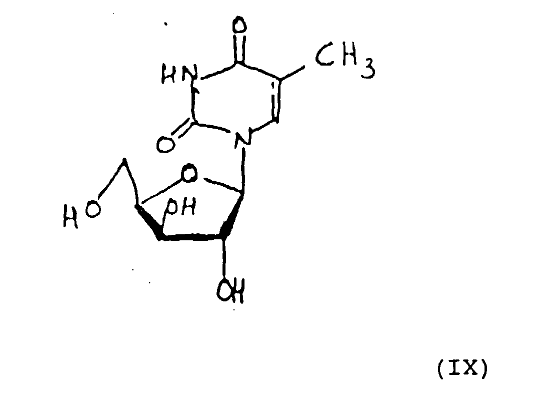 by removing, by conventional method, the acyl groups from 2, 3, 4-tri-0-acylxylothymidine of the formula (VIII) 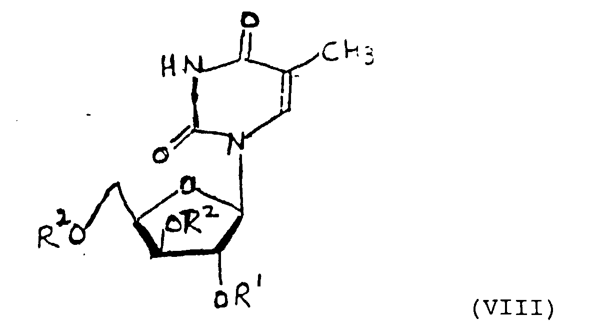 where R¹ represents acetyl group, R² represents benzoyl or acetyl group.
(b) condensing the deprotected xylothymidine of the formula (IX) with condensing agents like dialkyl or diaryl carbonates in the presence of a base and an organic solvent to yield 2,2'-anhydrothymidine of the formula (X). 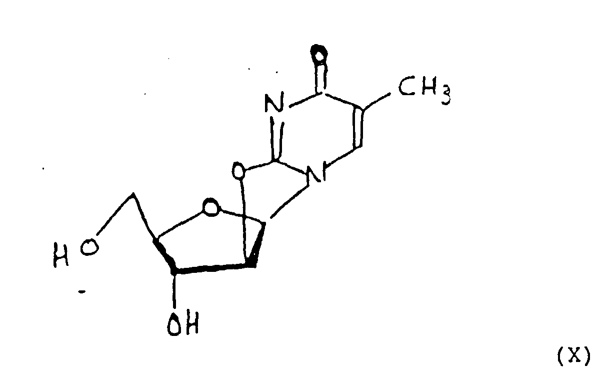
(c) brominating the 2,2'-anhydrothymidine of the formula (X) as defined above by conventional method to afford 2'-bromo derivative of the formula (XIV) 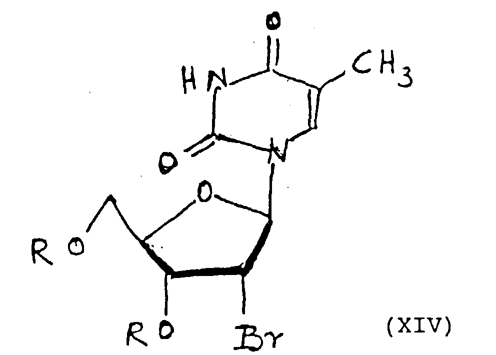 R, represents hydrogen or an acetyl group,
(d) reducing the 2'-bromo derivative of the formula (XIV) by conventional methods to yield beta-thymidine of the formula (II) or its diacetyl derivative and
(e) if desired deacylating the acetyl derivative of the formula (XV) 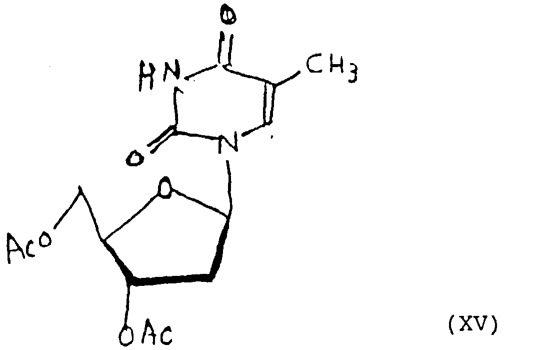 where Ac represents an acetyl group by conventional methods to yield Beta-thymidine of the formula (II).

The 1,2,3-tri-0-acylxlothymidine of the formula (VIII) can be prepared by condensing a mixture of alpha and beta-1,2,3,4-tetra-0-acylxylofuranose of the formula (VI) 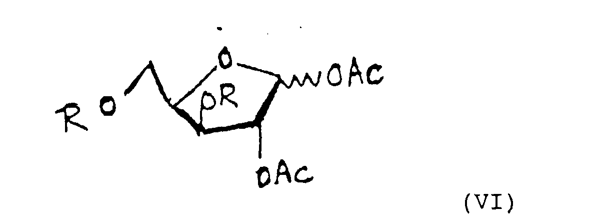 where R represents benzoyl group and Ac represents an acetyl group or by condensing a mixture of alpha and beta-1,2,3,4-tetra-0-acetylxylofuranose of the formula (VI) where R and Ac represent acetyl groups with 0,0-bis (trimethylsilyl) thymine of the formula (VII) 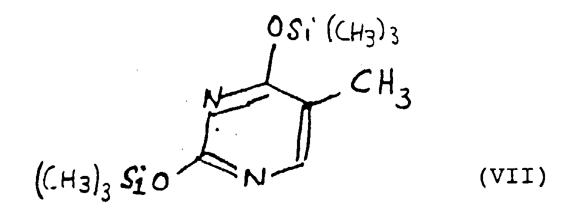 in the presence of condensing agents like Lewis acids.

The removal of acyl group from the compound of the formula (VI) can be effected in the presence of a base such as sodium methoxide, ammonia, potassium carbonate and the like. The organic solvent employed may be selected from methanol, ethanol and the like.

The condensation of the compound of formula (IX) may be effected using a condensing agent which may be selected from dialkyl or diaryl carbonate such as dimethyl, diethyl, diphenyl carbonate and the like. The condensation of the compound formula (IX) may be effected in the presence of base like sodium bicarbonate, sodium carbonate, potassium carbonate, and the like in the presence of polar nonprotic solvent agents like N,N-dimethylformamide. The condensation may be effected at a temperature in the range of 120 to 160°C.

The bromination of compound of formula (X) may be effected employing agents like hydrogen bromide dissolved in appropriate solvent like N,N-dimethylformamide and the like. The bromination may also be effected in the presence of agents like pyridinium hydrobromide, and the like.

The bromo derivative may be reduced employing usually employed agents such as Raney Nickel, palladium on charcoal and the like. The deacetylation of the compound of the formula (XV) may be effected using a base such as sodium methoxide, ammonia, and the like. Solvents such as methanol, ethanol and the like may also be employed for the deacetylation.

The known syntheses of beta-thymidine of the formula (II) are fraught with many difficulties, the foremost being expensive starting materials. For example beta-thymidine is prepared by the coupling reaction between protected 2-deoxy-D-ribose of the formula (III) 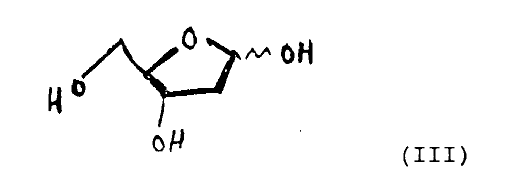 and protected thymine of the formula (VII) which unfortunately provides a mixture of alpha and beta nucleosides (A.J. Hubbard et al, Nucleic Acids Res. 1984, 12,682/; U. Nieball et al, J.Org. Chem. 1974, 39,3654; H. Vorbruggen et al, US Pat. No. 3,748,320).

The alpha-nucleoside has very little use in the synthesis of pharmaceutically useful compounds. Moreover the separation of alpha and beta nucleosides is a tedious process.

The recently reported process for the preparation of beta-thymidine comprises the coupling reaction between protected D-ribose of the formula (IV) where R represents acetyl group and protected thymine of the formula (VII) followed by deoxygenation at C-2'. This process, though it does not produce alpha nucleoside, is still an expensive process as it uses D-ribose as a starting material.

D-xylose of the formula (V) 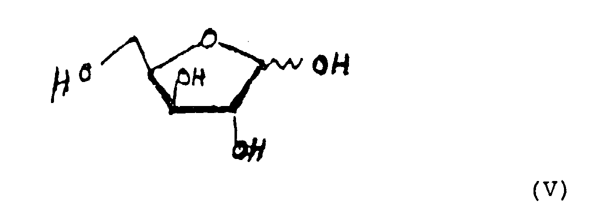 may be converted into a mixture of alpha and beta 1,2-di-O-acetyl-3-5-di-0-benzolyxylofuranose of the formula (VI) where R represents benzoyl group and Ac represents an acetyl group following the procedure described in J. Heterocyl, Chem. 1982, 19(3), 597,602. The condensation of compound of formula (VI) with thymine of the formula (VIIA), 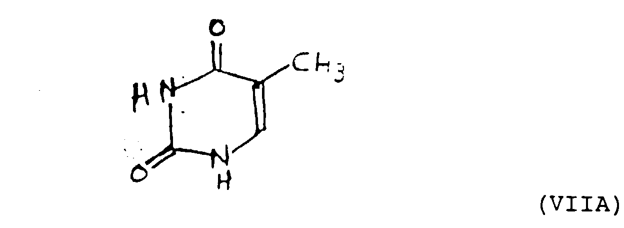 may be effected in the presence of tintetrachloride (SnCl₄), hexamethyldisilazane (HMDS) and trimethylsilychloride (TMSCl). Reference in this connection may be made to J. Med. Chem. 1986, 29 (2), 203-209. The coupling can also be done by condensing the above xylose derivative of the formula (VI) where R represents benzoyl and Ac represents an acetyl group with o,o-bis(trimethylsilyl) thymine of the formula (VII) in the presence of SnCl₄ - The removal of the acetyl or benzoyl groups from the resulting compound of formula (VIII) where R¹ represents an acetyl group and R² represents benzoyl group may be effected using base such as sodium alkoxide or ammonia in methanol or potassium carbonate in methanol to provide xylothymidine of the formula (IX).

Alternatively 1,2,3,5-tetra-O-acetylxylofuranose of the formula (VII) where R and Ac represent acetyl group is condensed with protected thymine of the formula (VII) and deacetylated under the conditions given above to provide xylothymidine of the formula (IX). The dialkyl or diaryl carbonates used for the condensation of xylothymidine may be dimethyl, diethyl or diphenylcarbonate. The condensation may be effected in the presence of sodium bicarbonate in N,N-dimethylformamide (DMF) at a temperature in the range of 120 - 160°C to give 2,2'-anhydrothymidine of the formula (X) which is acetylated with pyridime and acetyle anhydride to give 3',5'-di-o-acetyl-2,2'-anhydrothmidine of the formula (XI) 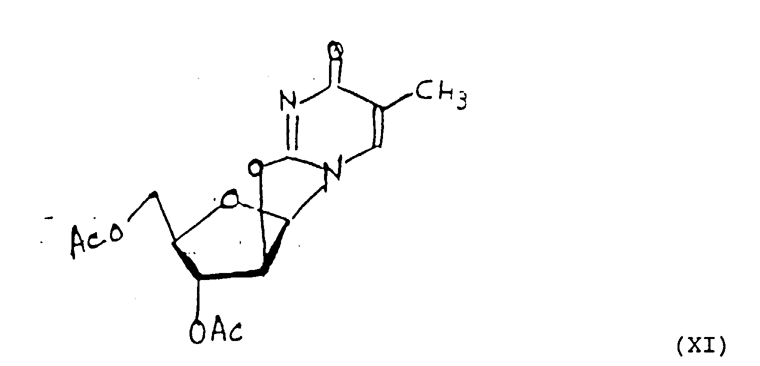 Ac represents acetyl group.

It is interesting to note that in the above reaction, a novel rearrangement via intermediates of the formula (XIII 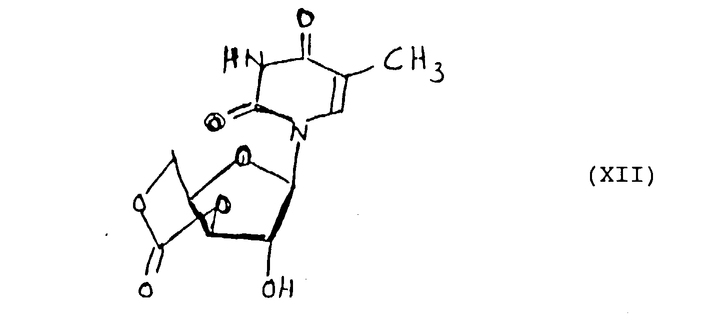 and (XIII)

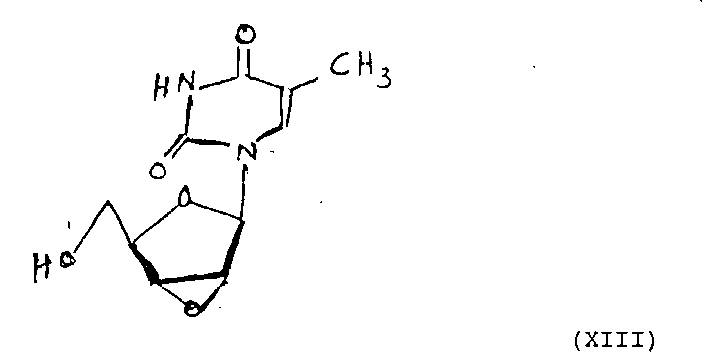

The treatment of 2,2'-anhydrothymidine of the formula (X) or its diacetyl derivative of the formula (XI) with hydrogen bromide in N,N-dimethyllormamide may be effected at temperature in the range of 90-110°C or with pyridinium-hydrobromide salt in pyridine at a temperature in the range of 110-130°C to afford the 2'bromo derivative of the formula (XIV) where R represent acetyl group. This compound is then reduced as such or as its diacetyl derivative in the presence of Raney Nickel or palladium on charcoal to afford beta-thymidine of the formula (II) or its diacetyl derivative of the formula (XV) and AC represents acetyl group. Deacetylation of the compound of the formula (XV) with mild base resulted in the formation of beta-thymidine of the formula (II).

The invention is described in detail in the examples given below which are provided to illustrate the invention only and therefore should not be construed to limit the scope of the present invention..

### Example 1:

### Converting an anomeric mixture of tetra-0-acetylxylofuranose of the formula (VI) to xylothymidine of the formula (IX).

To a solution of 35g (gm) of tetra-0-acetylribofuranose in 130 ml in a round bottom flask equipped with a magnetic stir bar and nitrogen inlet, was added 36g (gm) (1.2 eqv) of 0,0-bis(trimethylsilyl)thymine. To the slurry was added 14 ml (1.1 eqv) of tintetrachloride in 25 ml of dichloromethane. The resulting clear solution was stirred at room temperature for 18h (hrs), at the end of which thin layer chromatography (TLC) (2% CH₃OH in CHCl₃) showed no starting sugar. The reaction was quenched with 650 ml of saturated aqueous sodium bicarbonate solution. The mixture was filtered through celite to remove the tin salts. The organic layer, after separation was dried over sodium sulfate and concentrated to a light yellow foam (tri-O-acetylxylothymidine of the formula (VIII).

Crude tri-O-acetylxylothymidine (VIII) from the above reaction was dissolved in 300 ml of methanol and 0.525g (gm) (0.25 eqv) of sodium was added. After 8h (hrs). TLC (10% CH₃OH in CHCL₃) showed no starting triacetate. The reaction was quenched with Amberlite IR 120 resin^{®} and filtered. The filtrate was concentrated to afford 21.5g (gm) of xylothymidine of the formula (IX). (75% from tetra-O-acetylribofuranose).

### Example 2:

### Converting xylothymidine of the formula (IX) to 2,2-fanhydrothymidine of the formula (X)

A mixture of 10g (gm) of xylothymidine, 9.9g (gm) (1.2 eqv) of diphenylcarbonate and 300 mg of sodiumbicarbonate in 20 ml DMF was taken in a 250 ml round bottom flask equipped with a magnetic stirrer, reflux condenser and nitrogen inlet. The reaction mixture was heated to 150°C for 4h (hrs) at the end of which TLC (1:9 CH₃OH in ELOAc) showed no starting triol. The black solution was diluted with 10 ml of methanol and slowly added to 500 ml of ice cold benzone with stirring. The precipitated product was filtered, washed with benzene, dried and chromatographed (Eluent: 8% CH₃OG in CHCL₃) to yield 4.9g (gm) (53%) of anhydrothymidine.

### Example 3:

### Converting 2,2'-anhydrothymidine of the formula (X) to 3'5'-di-O-acetyl-2,2'-anhydrothymidine of the formula (XI)

To a solution of 4g (gm) of anhydrothymidine in 15 ml of pyridine was added 5.1g (gm) of acetic anhydride. The mixture was shifted at room temperature for 3h (hrs) when TLC (1:9 CH₃OH in EtOAc) showed no anhydrothymidine. The reaction was quenched with 10 ml of methanol and the solvents removed under vacuo. The brown semi solid is refluxed thrice in 50 ml benzene to yield 5.1g (gm) (94%) of 3'5'-di-O-acetyl-2,2'-anhydrothymidine of the formula (XI). m.p. 168-172°C.

### Example 4:

### Converting xylothymidine of the formula (IX) to 3',5'-di-O-acetyl-2,2'-anhydrothymidine of the formula (XI)

A 250 ml round bottom flask equipped with a magnetic stir bar, reflux condenser and nitrogen inlet was charged with 10g (gm) of xylothymidine of the formula (IX), 9.9g (gm) (1.2 eqv) of diphenyl carbonate.and 300 mg sodiumbicarbonate in 20 ml DMF was heated to 150°C for 4h (hrs) when TLC (1:9 CH₃OH in EtOAc) showed no starting xylothymidine. The reaction mixture was cooled and added dropwise into 600 ml ice cold benzene with stirring. The solid was filtered and dissolved in 30 ml pyridine. To the above stirred solution was added 12g (gm) acetic anhydride. After 3h (hrs). the reaction was quenched with 20 ml methanol, concentrated to dryness and the semi solid refluxed thrice in benzene (100 ml) to yield 4.8g (gm) of 3',5'-di-O-acetyl-2,2'-anhydrothymidine of the formula (XI) m.p. 168-171°C.

### Example 5:

### Converting 3',5'-di-O-acetyl 2,2'-anhydro thymidine of the formula (XI) to 2'-bromo-2'-deoxy-3'5'-di-O-acetylthymidine of the formula (XIV)

Diacetylanhydrothymidine of the formula (XI) (5g) was dissolved in 20 ml of DMF containing 1.5g (gm) (1.2 eqv) of anhydrous HBr and kept at 90-110°C for 2h (hrs). The reaction mixture was poured into 100 ml ice water, extracted thrice with chloroform (30 ml), the organic layers combined, dried and concentrated to yield 5.9g (gm) (95%) of 2'-bromo-2'-deoxy-3',5-di-O-acetylthymidine of the formula (XIV).

### Example 6

### Converting 2'-bromo-2'-deoxy-3', 5'-di-O-acetylthymidine of the formula (XIV) to beta-thymidine of the formula (II)

5g (gm) of 2'-bromo-2'-deoxy-3',5'-di-O-acetylthymdine of the formula (XIV) was hydrogenated in a Parr apparatus at 276 kPa (40 psi) in the presence of 5g (gm) Raney-Nickel in 30 ml methanol. After 4-5h (hrs) the reaction mixture was filtered through celite, concentrated, redissolved in 30 ml ethylacetate and washed thrice with water (30ml). The organic layer was dried over sodium sulfate and concentrated to give 3',5'-di-O-acetylthymidine of the formula (XV).

Crude 3',5'-di-O-acetylthymidine was dissolved in 20 ml methanol, and 0.075g (gm) sodium was added. After 8h (hrs) the reaction was quenched with Amberlite^{®} IR 120 resin, filtered and concentrated to yield beta-thymidine (2.4g (gm), 80%) m.p. 181-183°C.

## Claims

1. A process for the preparation of betathymidine of the formula (II) 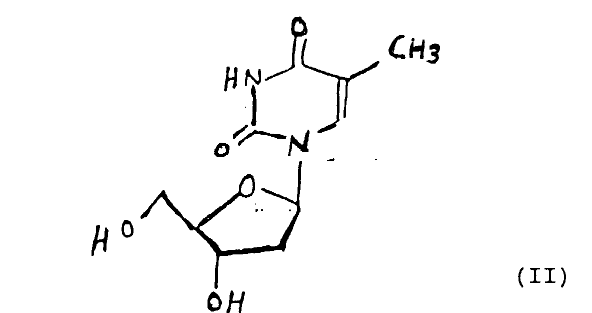 which comprises:
(a) preparing the deprotected xylothymidine of the formula (IX) 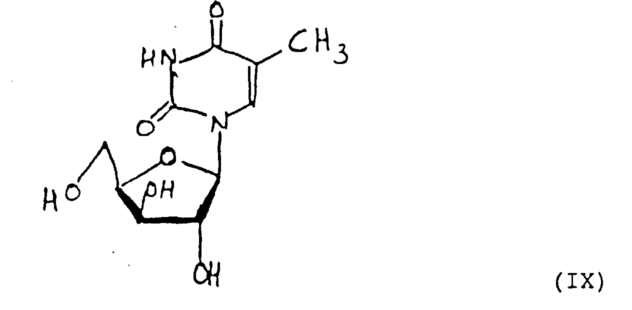 by removing by conventional method, the acyl groups from 1,2,3-tri-O-acyl-xylothymidine of the formula (VIII) 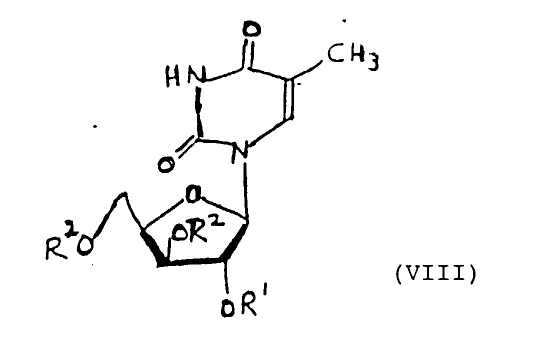 where R¹ represents an acetyl group, R² represents a benzoyl or an acetyl group.
(b) condensing the deprotected xylothymidine of the formula (IX) with dialkyl or diaryl carbonate in the presence of a base and an organic solvent to yield 2,2'-anhydrothymidine of the formula (X), 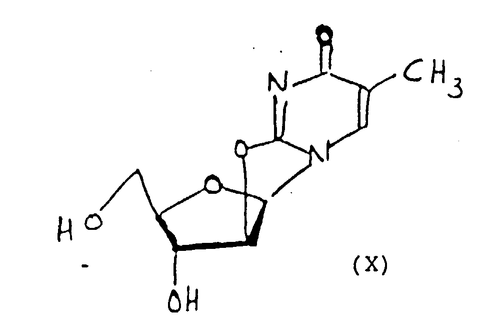
(c) brominating the 2,2'-anhydrothymidine of the formula (XI) 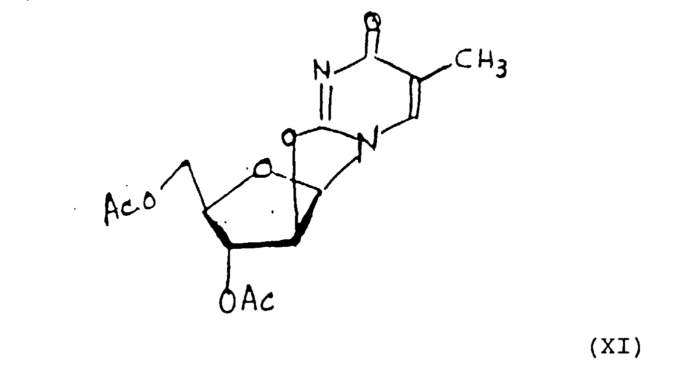 by conventional method to afford 2'-bromo derivative of the formula (XIV) 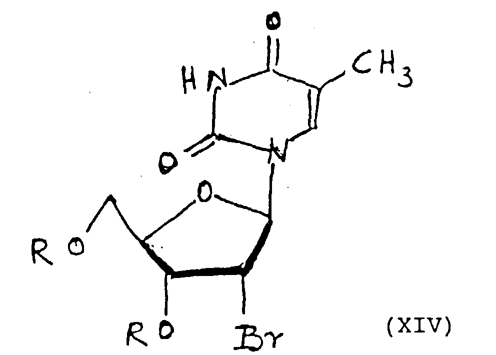 where R represents hydrogen or an acetyl group,
(d) reducing the 2'-bromo derivative of the formula (XIV) by conventional methods to yield beta-thymidine of the formula (II) or its diacetyl derivative of the formula (XV) 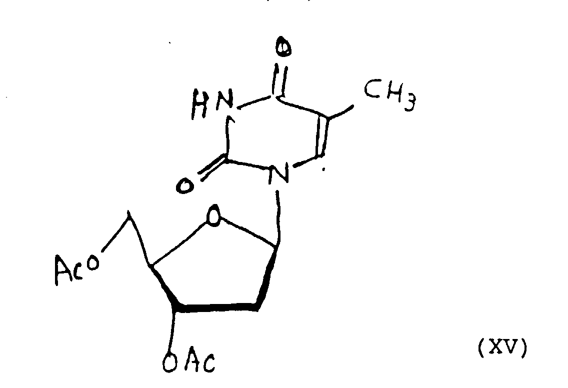 and
(e) if desired deacylating the acetyl derivative of the formula (XV) where Ac represents an acetyl group by conventional methods to yield beta-thymidine of the formula (II).

2. A process as claimed in claim 1 wherein the 1,2,3-tri-O-acyl-xylothymidine of the formula (VIII) is prepared by condensing a mixture of alpha and beta-1,2-di-O-acetyl-3,5-di-O-benzoyl xylofuranose of the formula (VI) 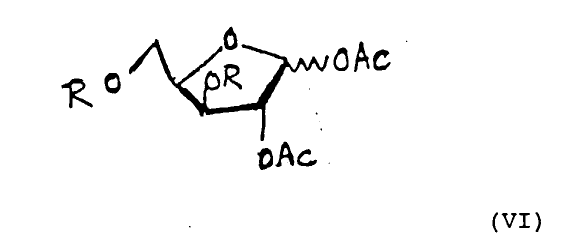 where R represents a benzoyl group and Ac represents an acetyl group or a mixture of alpha and beta-1,2,3,4-tetra-O-acetylxylofuranose of the formula (VI) where R and Ac represent acetyl groups with O,O-bis(trimethylsilyl)thymine of the formula (VII) 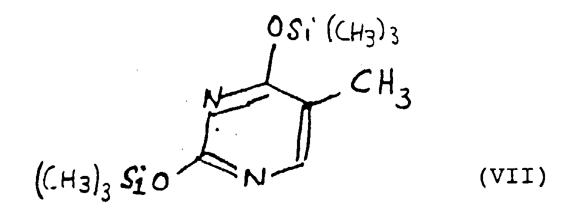 in the presence of a condensing agent.

3. A process as claimed in claim 2 wherein the condensing agent employed is a Lewis acid.

4. A process as claimed in claim 3 wherein the Lewis acid is SnCl₄.

5. A process as claimed in claim 1 wherein the removal of acyl group from the compound of the formula (VIII) is effected in the presence of a base selected from sodium methoxide, ammonia or potassium carbonate.

6. A process as claimed in claim 1 wherein the organic solvent employed is selected from methanol and ethanol.

7. A process as claimed in claim 1 wherein the dialkyl or diaryl carbonate employed for condensing the compound of formula (IX) is selected from dimethyl, diethyl or diphenyl carbonate.

8. A process as claimed in claim 1 wherein the condensation of the compound of the formula (IX) is effected in the presence of the base sodium bicarbonate.

9. A process as claimed in claim 1 wherein the condensation is effected in the presence of the polar nonprotic solvent N,N-dimethylformamide.

10. A process as claimed in claim 1 wherein the condensation is effected at a temperature in the range of 120 to 160°C.

11. A process as claimed in claim 1 wherein the bromination is effected employing hydrogen bromide.

12. A process as claimed in claim 1 wherein the bromination is effected in the presence of the brominating agent pyridinium hydrobromide.

13. A process as claimed in claim 1 wherein the solvents N,N-dimethylformamide, or pyridine are also used in the bromination step.

14. A process as claimed in claim 1 wherein the bromo derivative is reduced by employing Raney Nickel or palladium on charcoal.

15. A process as claimed in claim 1 wherein the deacetylation of the compound of the formula (XV) is effected using the base sodium methoxide or ammonia.

16. A process as claimed in claim 1 wherein the solvents methanol or ethanol are also employed for the deacetylation.

## Patentansprüche

1. Verfahren zur Herstellung von Betathymidin mit der Formel (II) 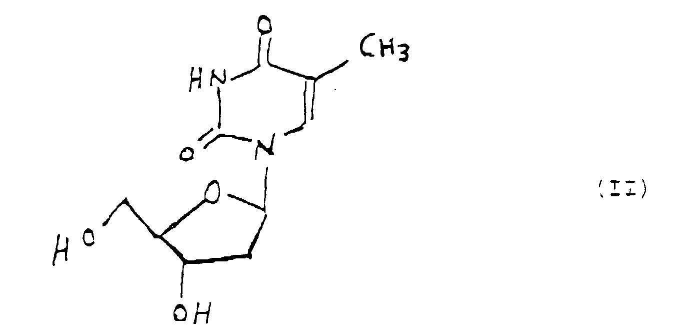 wobei es zu dem Verfahren gehört, dass:
(a) ein ungeschütztes Xylothymidin mit der Formel (IX) 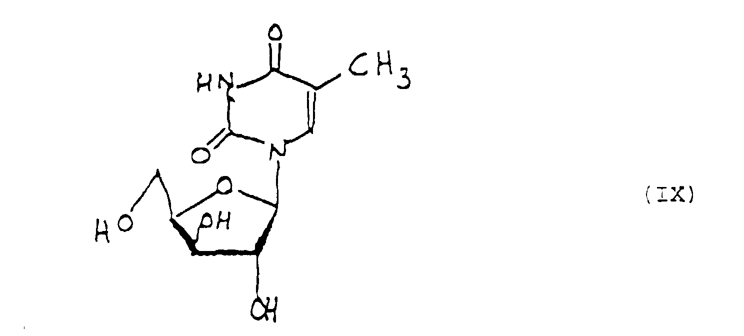 hergestellt wird, indem durch gebräuchliche Verfahren die Acylgruppen von dem 1,2,3-Tri-O-Acyl-Xylothymidin mit der Formel (VIII) 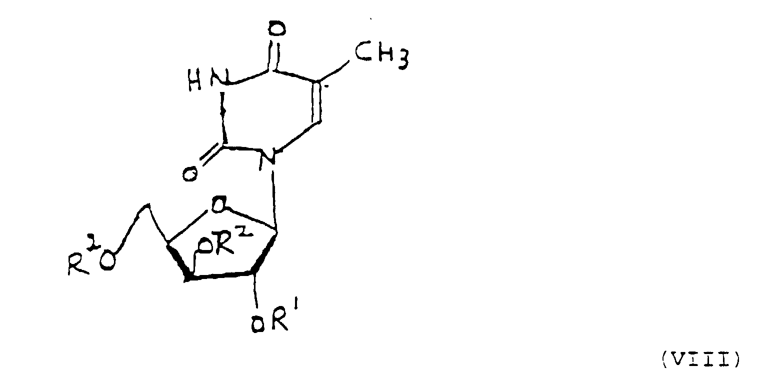 entfernt wird, wobei R¹ eine Acetylgruppe, R² eine Benzoyl- oder eine Acetylgruppe bedeuten,
(b) das ungeschützte Xylothymidin mit der Formel (IX) mit Dialkyl oder Diarylcarbonat in Anwesenheit einer 3ase oder einem organischen Lösungsmittel kondensiert wird, um 2,2'-Anhydrothymidin mit der Formal (X) zu enthalten, 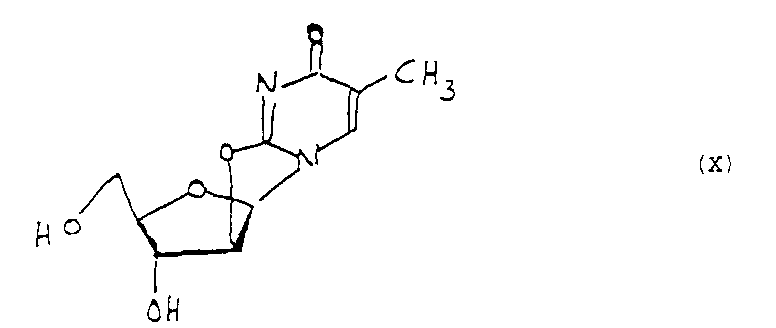
(c) das 2,2'-Anhydrothymidin mit der Formel (XI) 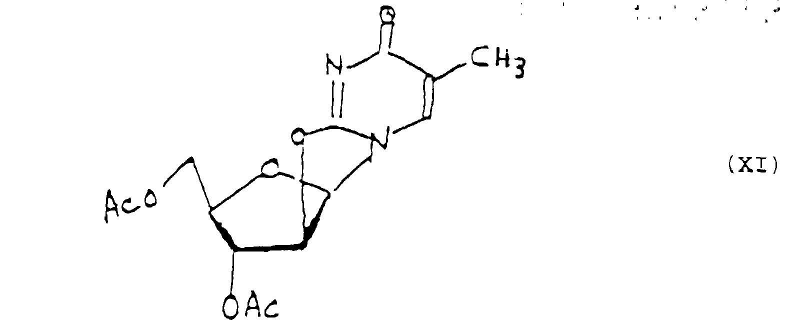 durch konventionelle Verfahren bromiert wird, um ein 2'-Bromoderivat mit der Formel (XIV) zu erhalten, 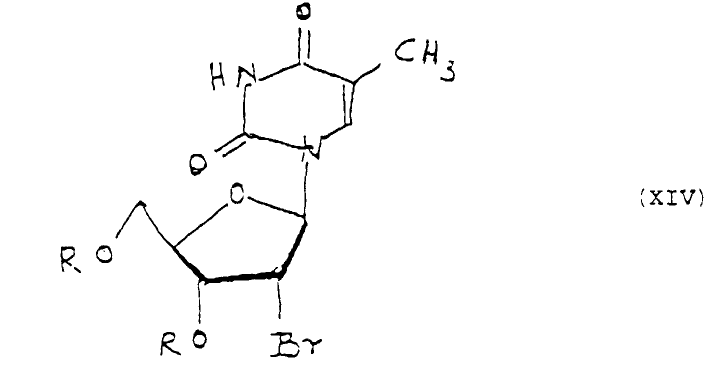 bei der R ein Wasserstoff oder eine Acetylgruppe bedeuten,
(d) das 2'-Bromderivat mit der Formal (XIV) durch konventionelle Verfahren reduziert wird, um Betathymidin mit der Formel (II) oder sein Diacetylderivat mit der Formel (XV) 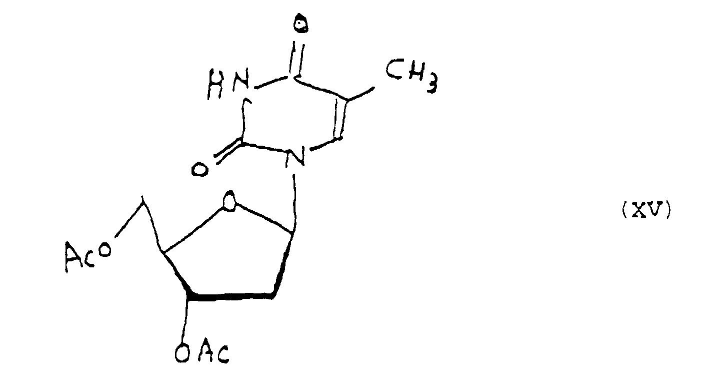 zu erhalten, und
(e) falls gewünscht, das Acetylderivat mit der Formel (XV) durch konventionelle Verfahren deaceliert wird, um Betathymidin mit der Formel (II) zu erhalten, wobei Ac eine Acetylgruppe bedeutet.

2. Verfahren nach Anspruch 1, wobei das 1,2,3-Tri-O-Acyl-Xylothimidin mit der Formel (VIII) durch Kondensation einer Mischung von Alpha- und Beta-1,2-di-O-Acetyl-3,5-di-O-Benzoylxylofuranose mit der Formel (VI) 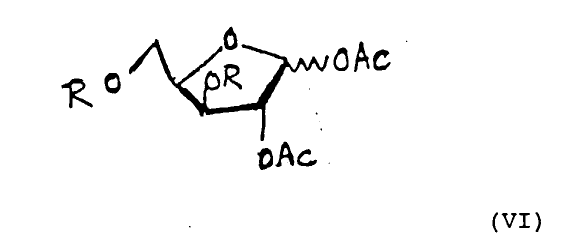 in Anwesenheit eines Kondensationswirkstoffes hergestellt wird, wobei R eine Benzoylgruppe und Ac eine Acetylgruppe oder eine Mischung aus einer Alpha- oder einer Beta-1,2,3,4-Tetra-O-Acetylxylofuranose mit der Formel (VI) bedeutet, wobei R und Ac Acetylgruppen mit O-O-bis (Trimethylsilyl) Thymin mit der Formel (VII)

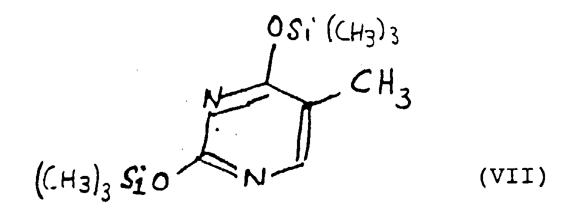

3. Verfahren nach Anspruch 2, bei dem als kondensierender Wirkstoff eine Lewis-Säure verwendet wird.

4. Verfahren nach Anspruch 3, bei dem die Lewis-Säure SnCl₄ ist.

5. Verfahren nach Anspruch 1, bei dem das Entfernen der Acylgruppe aus der Verbindung mit der Formel (VIII) in Anwesenheit einer Base ausgeführt wird, die aus einer Gruppe ausgewählt ist, zu der Natriummethoxid, Ammoniak oder Kaliumkarbonat gehören.

6. Verfahren nach Anspruch 1, bei dem das verwendete organische Lösungsmittel aus einer Gruppe ausgewählt ist, zu der Methanol und Ethanol gehören.

7. Verfahren nach Anspruch 1, bei dem das Dialkyloder Diarylkarbonat, das zum Kondensieren der Verbindung mit der Formel (IX) verwendet wird, aus einer Gruppe ausgewählt ist, zu der Dimethyl-, Diethyl- oder Diphenylkarbonat gehören.

8. Verfahren nach Anspruch 1, bei dem das Kondensieren der Verbindung mit der Formel (IX) in Anwesenheit der Base Natriumbikarbonat ausgeführt wird.

9. Verfahren nach Anspruch 1, bei dem das Kondensieren in Anwesenheit des polaren, nichtprotischen Lösungsmittels N,N-Dimethylformamid bewirkt wird.

10. Verfahren nach Anspruch 1, bei dem das Kondensieren bei einer Temperatur im Bereich zwischen 120° und 160° C bewirkt wird.

11. Verfahren nach Anspruch 1, bei dem das Bromieren unter Verwendung von wasserstoffbromid bewirkt wird,

12. Verfahren nach Anspruch 1, bei dem das 3romieren in Anwesenheit des bromierenden Wirkstoffes Pyridinhydrobromid bewirkt wird.

13. Verfahren nach Anspruch 1, bei dem die Lösungsmittel N,N-Dimethylformamid oder Pyridin auch in dem Bromierungsschritt verwendet werden.

14. Verfahren nach Anspruch 1, bei dem das Bromderivat reduziert wird, indem Raney-Nickel oder Palladium auf Holzkohle verwendet wird.

15. Verfahren nach Anspruch 1, bei dem die Deacetylierung der Verbindung mit der Formel (XV) unter Verwendung der Base wie Natriummethoxid oder Ammoniak bewirkt wird.

16. Verfahren nach Anspruch 1, bei dem die Lösungsmittel Methanol oder Ethanol auch bei der Deacetylierung verwendet werden.

## Revendications

1. Procédé pour la préparation de bêta-thymidine de formule (II) 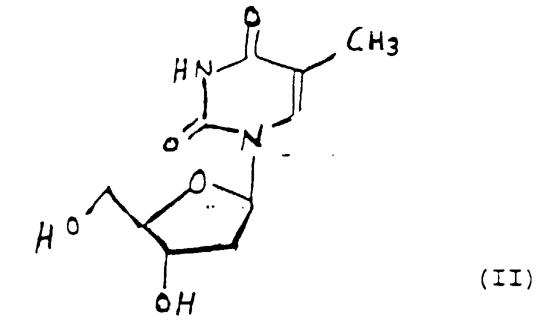 qui comprend :
(a) la préparation de la xylothymidine déprotégée, de formule (IX) 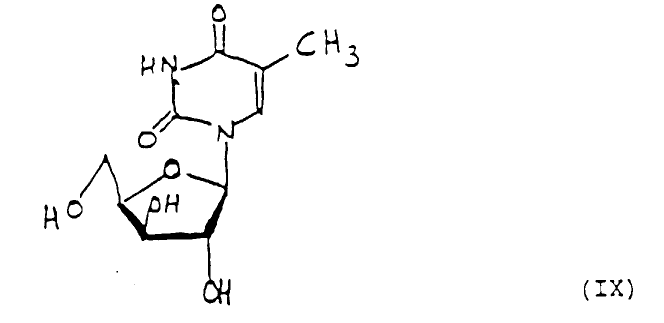 en éliminant par une méthode classique les groupes acyle de la 1,2,3-tri-O-acyl-xylothymidine de formule (VIII) 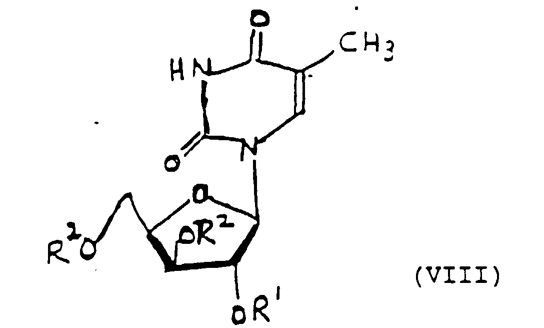 où R¹ représente un groupe acétyle, R² représente un groupe benzoyle ou un groupe acétyle,
(b) condensation de la xylothymidine déprotégée de formule (IX) avec un carbonate de dialkyle ou diaryle en présence d'une base et d'un solvant organique pour obtenir la 2,2'-anhydrothymidine de formule (X) 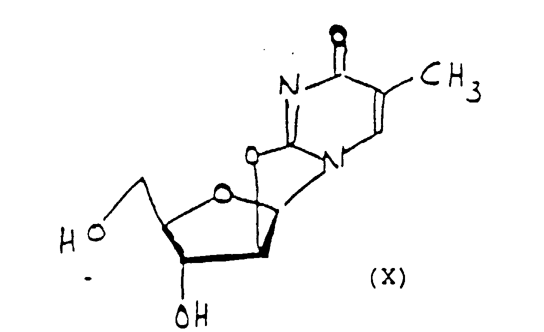
(c) la bromation de la 2,2'-anhydrothymidine de formule (XI) 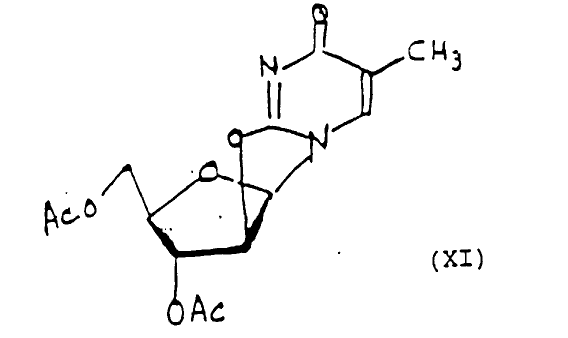 par une méthode classique pour obtenir le dérivé 2'-bromé de formule (XIV) 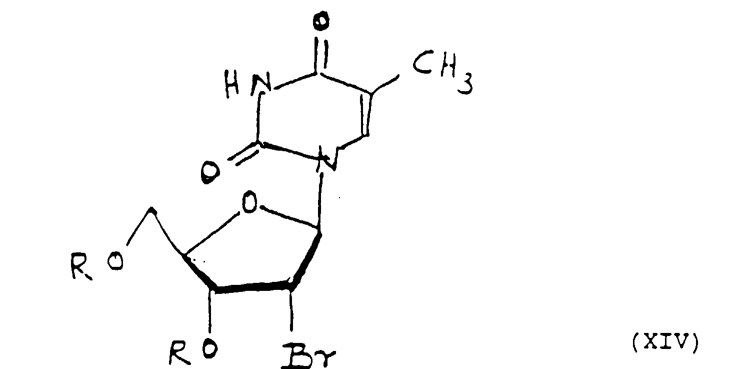 où R représente l'hydrogène ou un groupe acétyle,
(d) la réduction du dérivé 2'-bromé de formule (XIV) par des méthodes classiques pour obtenir la bêta-thymidine de formule (II) ou son dérivé diacétylé de formule (XV) 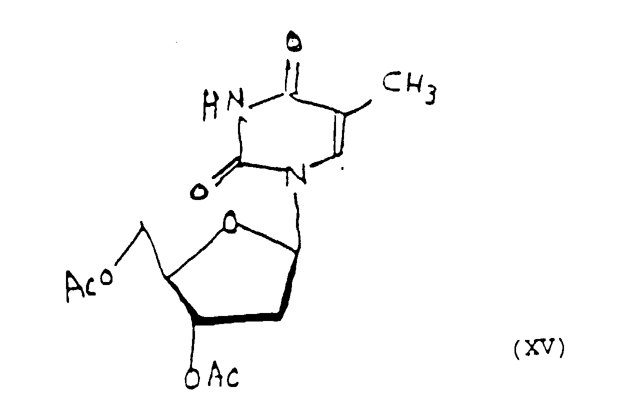 et
(e) facultativement, la désacyclation du dérivé acétylé de formule (XV), où Ac représente un groupe acétyle, par des méthodes classiques pour obtenir la bêta-thymidine de formule (II).

2. Procédé tel que revendiqué dans la revendication 1, dans lequel la 1,2,3-tri-O-acyl-xylothymidine de formule (VIII) est préparée en condensant un mélange d'alpha- et bêta-1,2-di-O-acétyl-3,5-di-O-benzoyl-xylo-furannoses de formule (VI) 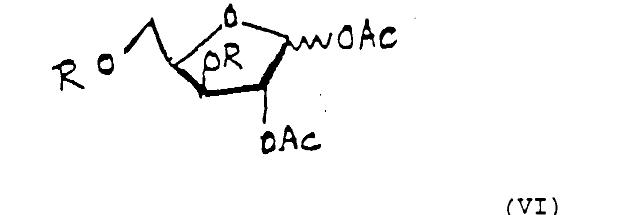 où R représente un groupe benzoyle et Ac représente un groupe acétyle, ou un mélange d'alpha- et bêta-1,2,3,4-étra-O-acétylxylofurannoses de formule (VI) où R et Ac représentent des groupes acétyle avec la O,O-bis(triméthylsilyl)thymine de formule (VII) 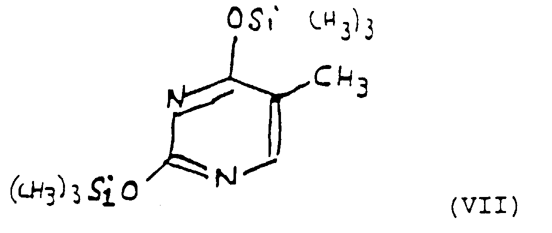 en présence d'un agent de condensation.

3. Procédé tel que revendiqué dans la revendication 2, dans lequel l'agent de condensation utilisé est un acide de Lewis.

4. Procédé tel que revendiqué dans la revendication 3, dans lequel l'acide de Lewis est SnCl₄.

5. Procédé tel que revendiqué dans la revendication 1, dans lequel l'élimination des groupes acyle du composé de formule (VIII) est effectuée en présence d'une base choisie parmi le méthylate de sodium, l'ammoniac ou le carbonate de potassium.

6. Procédé tel que revendiqué dans la revendication 1, dans lequel le solvant organique utilisé est choisi parmi le méthanol et l'éthanol.

7. Procédé tel que revendiqué dans la revendication 1, dans lequel le carbonate de dialkyle ou diaryle utilisé pour condenser le composé de formule (IX) est choisi parmi les carbonates de diméthyle, diéthyle ou diphényle.

8. Procédé tel que revendiqué dans la revendication 1, dans lequel la condensation du composé de formule (IX) est effectuée en présence de bicarbonate de sodium comme base.

9. Procédé tel que revendiqué dans la revendication 1, dans lequel la condensation est effectuée en présence de N,N-diméthylformamide comme solvant aprotique polaire.

10. Procédé tel que revendiqué dans la revendication 1, dans lequel la condensation est effectuée à une température comprise dans l'intervalle de 120 à 160°C.

11. Procédé tel que revendiqué dans la revendication 1, dans lequel la bromation est effectuée en utilisant du bromure d'hydrogène.

12. Procédé tel que revendiqué dans la revendication 1, dans lequel la bromation est effectuée en présence d'hydrobromure de pyridinium comme agent de bromation.

13. Procédé tel que revendiqué dans la revendication 1, dans lequel les solvants N,N-diméthylformamide ou pyridine sont également utilisés dans l'étape de bromation.

14. Procédé tel que revendiqué dans la revendication 1, dans lequel le dérivé bromé est réduit en utilisant du nickel de Raney ou du palladium sur charbon.

15. Procédé tel que revendiqué dans la revendication 1, dans lequel la désacétylation du composé de formule (XV) est effectuée en utilisant le méthylate de sodium ou l'ammoniac comme base.

16. Procédé tel que revendiqué dans la revendication 1, dans lequel les solvants méthanol ou éthanol sont également utilisés pour la désacétylation.
